# EUROPEAN PATENT APPLICATION

(11) **EP 4 586 346 A1**
(43) Date of publication of application: **16.07.2025**
(21) Application number: 25150558.2
(22) Date of filing: 07.01.2025
(51) Int. Cl.: H01M 8/04029, H01M 8/04007, H01M 8/0612, H01M 8/249, H01M 8/18, H01M 8/12, C07C 1/12, C07C 9/04, C07C 1/00, C25B 1/042, C25B 15/08

(54) **USE OF SOLID OXIDE CELLS IN A METHANE PRODUCTION PROCESS AND AN ELECTRICITY GENERATION PROCESS**

(30) Priority: 09.01.2024 NL 2036765
(71) Applicant: van Putten, Arnold Jan, 5644 BX Eindhoven (NL); Philips, Danny Maria Hubertus, 5691 LG Son en Breugel (NL)
(72) Inventor: van Putten, Arnold Jan, 5644 BX Eindhoven (NL); Philips, Danny Maria Hubertus, 5691 LG Son en Breugel (NL)
(74) Representative: Dekker-Garms, Alwine Emilie

(57) **Abstract**

In the field of using methane as an energy storage medium, wherein electricity is used at one stage for producing methane, and wherein methane is used at a later stage for generating electricity, use is made of solid oxide cells. The solid oxide cells are combined to stacks, the stacks are arranged in units (10), and the units (10) are arranged in modules (9). Each of the modules (9) is included in a subsystem (29) and is arranged in a frame (30) of the subsystem (29). Finally, a number of subsystems (29) are combined to a system. In this way, costs of the electricity to be generated are reduced. Further reduction of the costs is achieved by having components which are shared between the modules (9) and the subsystems (29), particularly components involved in supplying and discharging fluids and components involved in reusing heat of output fluid flows.

## Description

The invention relates to a module configured for application in a methane production process comprising use of electricity in producing methane on the basis of steam and carbon dioxide, and also for application in an electricity generation process comprising use of methane in generating electricity and producing carbon dioxide and steam, the module comprising a solid oxide unit comprising at least one solid oxide stack of at least two solid oxide cells, wherein each of the solid oxide cells comprises a component configured to function as anode during operation of the solid oxide cell, a component configured to function as cathode during operation of the solid oxide cell, and a solid oxide component configured to function as electrolyte during operation of the solid oxide cell.

The invention further relates to a subsystem comprising a frame and at least two modules mentioned here before, wherein the modules are arranged in the frame. The invention also relates to a system comprising at least two subsystems of the type mentioned here before.

In the field of generating and using energy, it is an ongoing trend to rely less on the use of fossil fuels, and it is even intended to eventually terminate the use of fossil fields. Upcoming fields include the field of generating electricity on the basis of wind power, the field of generating electricity by means of solar cells, and the field of generating electricity on the basis of water motion. One of the issues which need to be dealt with in such upcoming fields is that when environmental conditions are optimal for generating electricity, it may happen that more electricity is generated than needed, and that when environmental conditions are less than optimal for generating electricity, it may happen that the energy which is generated at that time, if any, is not enough for fulfilling the need. A solution to this mismatch of demand and supply is found in storage of energy.

The invention is in the particular field of using methane as an energy storage medium, wherein electricity, which may be excess electricity in a non-fossil system, is used at one stage for producing methane, and wherein methane is used at a later stage for generating electricity, when more electricity is needed than can be generated by the non-fossil system at that later stage. In practical applications, the methane is injected into an existing gas network at the one stage, and is retrieved from the same existing gas network at the later stage.

It is known to use a so-called solid oxide cell in the above-mentioned processes of producing methane and generating electricity, which in the present text are referred to as methane production process and electricity generation process, respectively. Among other things, a solid oxide cell comprises a component configured to function as anode during operation of the solid oxide cell, a component configured to function as cathode during operation of the solid oxide cell, and a solid oxide component configured to function as electrolyte during operation of the solid oxide cell. A solid oxide cell is configured to produce hydrogen (H₂) on the basis of a supply of electricity and gases including steam in the methane production process, and to generate electricity on the basis of a supply of gases including methane in the electricity generation process, and to also output carbon dioxide and steam in the same process. Further, it is known to use a catalyst unit with the solid oxide cell, which catalyst unit includes a metal catalyst such as a nickel catalyst. In the methane production process, the hydrogen which is produced by the solid oxide cell is mixed with carbon dioxide (CO₂), and the mixture is enabled to interact with the catalyst, as a result of which the methane is obtained.

In order to operate the solid oxide cell in a proper and efficient manner, it is practical if the solid oxide cell is used with means for providing the solid oxide cell with gas mixtures containing respective gases in defined percentages, and if the temperature of the solid oxide cell is controlled to be in a defined range, such as a range of relatively high temperatures of about 650°C to 750°C, a range of lower temperatures of about 400°C to 600°C, or another range as appropriate in view of the type of solid oxide cell which is used. It is an objective of the invention to provide a way of putting to practice the above-described technology aimed at producing methane at one stage and using the methane in a process of generating electricity at a later stage, such that the costs of the electricity to be generated can be acceptable against a background of other available technologies of generating electricity, and commercial exploitation of the invention is feasible.

In view of the foregoing, the invention provides a module configured for application in a methane production process comprising use of electricity in producing methane on the basis of steam and carbon dioxide, and also for application in an electricity generation process comprising use of methane in generating electricity and producing carbon dioxide and steam, the module comprising:
- a solid oxide unit comprising at least one solid oxide stack of at least two solid oxide cells, wherein each of the solid oxide cells comprises a component configured to function as anode during operation of the solid oxide cell, a component configured to function as cathode during operation of the solid oxide cell, and a solid oxide component configured to function as electrolyte during operation of the solid oxide cell;
- a first heat exchanger arranged and configured to enable exchange of heat between output air of the solid oxide unit and water originating from a water supply to the module during the electricity generation process;
- a second heat exchanger arranged and configured to enable exchange of heat between an output mixture of the solid oxide unit comprising steam and hydrogen, and gaseous fuel comprising methane originating from a methane supply to the module and hydrogen during the electricity generation process;
- a third heat exchanger arranged and configured to enable exchange of heat between output air of the solid oxide unit and input air originating from an air supply to the module during both the methane production process and the electricity generation process, and to pass the input air on to the solid oxide unit;
- a mixing and dosing unit arranged and configured to receive steam originating from a steam source and also output hydrogen of the solid oxide unit during the methane production process, to receive steam from the first heat exchanger and also the gaseous fuel from the second heat exchanger during the electricity generation process, and to output a mixed flow during both the methane production process and the electricity generation process; and
- a fourth heat exchanger arranged and configured to enable exchange of heat between the output mixture of the solid oxide unit and an output mixed flow of the mixing and dosing unit during both the methane production process and the electricity generation process, and to pass the output mixed flow of the mixing and dosing unit on to the solid oxide unit.

It follows from the foregoing definition that the invention involves using more than one solid oxide cell. In particular, at least two solid oxide cells are combined to a solid oxide stack, and at least one solid oxide stack is included in a solid oxide unit, wherein the solid oxide unit is part of a module which further comprises a number of heat exchangers and a mixing and dosing unit. The heat exchangers have a function in extracting heat from output flows of the solid oxide unit and supplying the heat to input flows of the solid oxide unit, and the mixing and dosing unit has a function in making a mixture of gases to be supplied to the solid oxide unit.

Use in a module of at least one solid oxide unit comprising at least one solid oxide stack of at least two solid oxide cells offers a possibility of choosing a number of solid oxide units, solid oxide stacks per solid oxide unit and solid oxide cells per solid oxide stack in order to realize a desired power level of the module which may be considerably higher than the power level of a single solid oxide cell and may even be considerably higher than the power level of a single solid oxide unit. Further, use in a module of at least one solid oxide unit comprising at least one solid oxide stack of at least two solid oxide cells offers a possibility of realizing a configuration of the module in which a number of tubes and other components is kept to a minimum by assigning the tubes and other components to more than one solid oxide cell, possibly to more than one solid oxide stack, and possibly even to more than one solid oxide unit. Extracting heat from output flows of the solid oxide unit and supplying the heat to input flows of the solid oxide unit is a way of saving on external supply of energy, wherein measures can be taken to keep a loss of heat through outlet flows to a minimum. Having a mixing and dosing unit involves a possibility of making a mixture of gases according to specifications, so that the solid oxide cell can be operated under conditions which are beneficial to the functionality and efficiency of the solid oxide cell. In the context of the module, a practical example of the output mixed flow of the mixing and dosing unit during the methane production process is a flow comprising steam and hydrogen, and a practical example of the output mixed flow of the mixing and dosing unit during the electricity generation process is a flow comprising steam, hydrogen and methane.

In short, a first heat exchanger is functional to recover heat from output air of the solid oxide unit, which heat is used to heat input water during the electricity generation process, a second heat exchanger is functional to recover heat from an output gas mixture of the solid oxide unit, which heat is used to heat input fuel during the electricity generation process, a third heat exchanger is functional to recover heat from output air of the solid oxide unit, which heat is used to heat input air during both the methane production process and the electricity generation process, and a fourth heat exchanger is functional to recover heat from an output gas mixture of the solid oxide unit, which heat is used to heat an input gas mixture during both the methane production process and the electricity generation process. In this way, all main output flows and all main input flows of the respective solid oxide cells are involved in reuse of heat. It is practical if the module is equipped with more heat exchangers than the ones mentioned here before, and probably also with heaters at strategic positions.

In a practical embodiment of the module, the first heat exchanger comprises a tube system for transport of water and creation of steam. It is advantageous if bodies which are functional to increase a heat exchanging surface of the tube system are mounted on at least a portion of the tube system, because this is a measure for promoting the exchange of heat between the water in the tube system and the output air of the solid oxide unit. Likewise, it is practical if the second heat exchanger comprises a tube system for transport of the gaseous fuel, and if bodies which are functional to increase a heat exchanging surface of the tube system are mounted on at least a portion of the tube system.

There is no need for an external supply of hydrogen to the module. Instead, hydrogen which is output by the solid oxide unit can be used for mixing with the methane supplied to the mixing and dosing unit during the electricity generation process.

For the purpose of enabling the actual production of methane during the methane production process, it is practical if the module is equipped with a catalyst unit including a catalyst, and if the catalyst unit is arranged and configured to receive a mixture of hydrogen originating from the solid oxide unit and carbon dioxide during the methane production process, and to enable interaction between the catalyst and the mixture of hydrogen and carbon dioxide.

The invention further provides a subsystem comprising a frame and at least two modules as defined and described in the foregoing, wherein the modules are arranged in the frame. By making a combination of at least two modules, an even higher power level can be realized, and also a further reduction of costs of the electricity to be generated can be achieved when components are shared between the modules.

In a practical embodiment of the subsystem, the frame is shaped like a matrix and is designed to position at least four modules in at least two columns. In that case, it is possible that the frame is designed to have a staggered configuration of the columns of the modules. This is an advantageous way of achieving that sufficient space is present at a bottom side of the frame for accommodating tube systems of adjacent columns extending to a same side of the frame. For example, tube systems for discharging gas from a row of modules are generally straight and are in stacked configuration in column direction. Enabling relatively simple shapes of the respective tube systems is another cost-saving measure.

An example of a shared component in the subsystem is an air discharge channel. In particular, an embodiment of the subsystem is feasible in which the subsystem comprises a single air discharge channel in fluid communication with the modules for receiving air from the modules during both the methane production process and the electricity generation process, and suction means to actively extract air at the position of the air discharge channel. It is an insight of the invention that by creating underpressure in the discharge channel, it is achieved that input of air can take place at a relatively low pressure, which is advantageous in view of the fact that the solid oxide cells usually comprise very thin ceramic sheets and are thereby sensitive to pressure differences.

It is further possible that the subsystem comprises a single air supply channel in fluid communication with the modules for supplying air to the modules during both the methane production process and the electricity generation process, and blower means to exert pressure on the air and to put the air in motion towards the modules at the position of the air supply channel. In conformity with earlier explanations, it is noted that having a shared air supply channel and shared blower means constitutes a cost-reducing factor.

Yet another example of a component which can be shared between the modules is a single hydrogen discharge channel in fluid communication with the modules for receiving hydrogen from the modules during the methane production process.

The invention also provides an even larger entity, namely a system comprising at least two subsystems as defined and described in the foregoing. In the context of such a system, it is a feasible option that a single steam vessel in fluid communication with the modules of the subsystems is used for supplying steam to the modules of the subsystems during the methane production process.

Further, it is disclosed that it is advantageous to have a subsystem comprising a frame and at least two modules configured for application in a methane production process comprising use of electricity in producing methane on the basis of steam and carbon dioxide, and also for application in an electricity generation process comprising use of methane in generating electricity and producing carbon dioxide and steam, wherein each of the modules comprises a solid oxide unit comprising at least one solid oxide stack of at least two solid oxide cells, wherein each of the solid oxide cells comprises a component configured to function as anode during operation of the solid oxide cell, a component configured to function as cathode during operation of the solid oxide cell, and a solid oxide component configured to function as electrolyte during operation of the solid oxide cell, and wherein the modules are arranged in the frame.

Options relating to the subsystem include the following:
- the frame is shaped like a matrix and is designed to position at least four modules in at least two columns;
- the frame is designed to have a staggered configuration of the columns of the modules;
- tube systems for discharging gas from a row of modules are generally straight and are in stacked configuration in column direction;
- the subsystem comprises a single air discharge channel in fluid communication with the modules for receiving air from the modules during both the methane production process and the electricity generation process, and suction means to actively extract air at the position of the air discharge channel;
- the subsystem comprises a single air supply channel in fluid communication with the modules for supplying air to the modules during both the methane production process and the electricity generation process, and blower means to exert pressure on the air and to put the air in motion towards the modules at the position of the air supply channel; and
- the subsystem comprises a single hydrogen discharge channel in fluid communication with the modules for receiving hydrogen from the modules during the methane production process.

The subsystem may be part of a system comprising at least two of the subsystems. It is practical if such a system comprises a single steam vessel in fluid communication with the modules of the subsystems for supplying steam to the modules of the subsystems during the methane production process.

Still further, it is disclosed that it is advantageous to have a module configured for application in a methane production process comprising use of electricity in producing methane on the basis of steam and carbon dioxide, and also for application in an electricity generation process comprising use of methane in generating electricity and producing carbon dioxide and steam, the module comprising a solid oxide unit comprising at least one solid oxide stack of at least two solid oxide cells, wherein each of the solid oxide cells comprises a component configured to function as anode during operation of the solid oxide cell, a component configured to function as cathode during operation of the solid oxide cell, and a solid oxide component configured to function as electrolyte during operation of the solid oxide cell, and the module further comprising a heat exchanger arranged and configured to enable exchange of heat between fluid flows in the module, wherein the heat exchanger comprises a tube system for transport of a fluid flow, and wherein bodies which are functional to increase a heat exchanging surface of the tube system are mounted on at least a portion of the tube system.

The invention will now be explained in greater detail with reference to the drawings, in which equal or similar parts are indicated by the same reference signs, and in which:
Figure 1 illustrates use of a solid oxide stack of solid oxide cells in a methane production process;
Figure 2 illustrates use of the same solid oxide stack in an electricity generation process;
Figures 3 and 4 show a scheme of components of a module including a solid oxide unit, wherein figure 3 illustrates use of the components in the methane production process, and wherein figure 4 illustrates use of the components in the electricity generation process;
Figure 5 illustrates a practical option in respect of a general set-up of a mixing and dosing unit of the module, wherein flows through the mixing and dosing unit are indicated in respect of both the methane production process and the electricity generation process;
Figures 6 and 7 diagrammatically show a front view and a side view, respectively, of the module;
Figure 8 diagrammatically shows a top view of a section of the module at the position of heat exchanger tubes extending in the module;
Figure 9 diagrammatically shows a front view of a subsystem comprising a frame and twelve modules arranged in the frame in three columns of four modules;
Figures 10 and 11 diagrammatically show a side view and a front view, respectively, of the frame of the subsystem;
Figures 12 and 13 illustrate how thermal insulation sheets can be used to cover the subsystem;
Figure 14 diagrammatically shows a top view of a section of the frame and the thermal insulation sheets; and
Figure 15 diagrammatically shows a top view of a system comprising a steam vessel and eight subsystems arranged in two groups of four subsystems at opposite sides of the steam vessel.

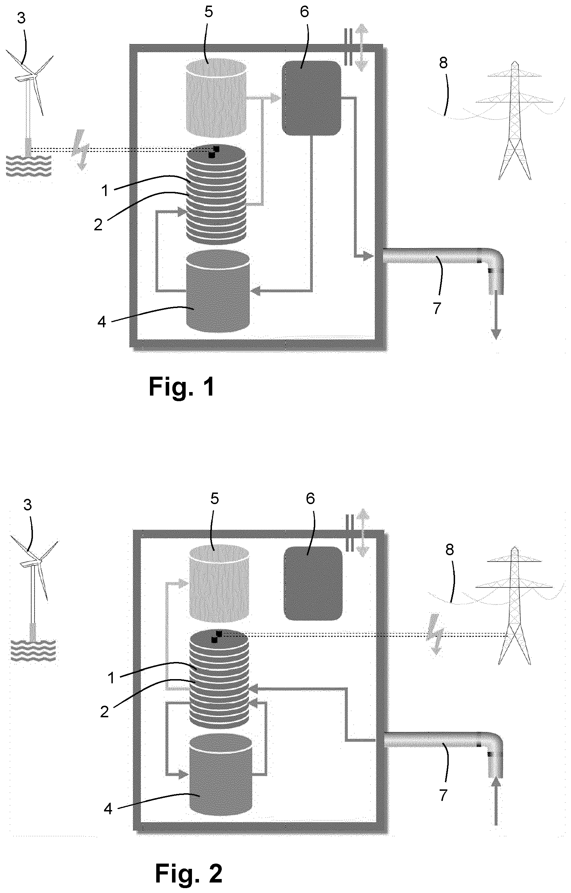

Figure 1 illustrates use of a solid oxide stack 1 of solid oxide cells 2 in a methane production process, and figure 2 illustrates use of the same solid oxide stack 1 in an electricity generation process. A solid oxide cell 2 is known in the art. Generally speaking, the solid oxide cell 2 comprises a component configured to function as anode during operation of the solid oxide cell 2, a component configured to function as cathode during operation of the solid oxide cell 2, and a solid oxide component configured to function as electrolyte during operation of the solid oxide cell 2. In a known embodiment, the solid oxide cell 2 comprises thin ceramic sheets containing nickel oxide. The number of solid oxide cells 2 in a solid oxide stack 1 can be quite large, such as a number in a range of seventy to a hundred.

In the methane production process, electricity is supplied to the solid oxide stack 1 from an electricity source 3 such as a wind turbine, and steam is supplied to the solid oxide stack 1 from a steam source 4, while the solid oxide stack 1 is at a temperature in a range of temperatures of about 650°C to 750°C. As a result, the solid oxide stack 1 produces hydrogen. The hydrogen is mixed with carbon dioxide supplied by a source 5 of carbon dioxide, and the gas mixture thus obtained is supplied to a catalyst unit 6 including a catalyst such as a nickel catalyst. In the catalyst unit 6, methane is produced. Steam is also output by the catalyst unit 6. The steam is condensed and the water thus obtained is optionally fed to the steam source 4. The methane is injected into an existing gas network 7 and transported to a storage area. In the methane production process, air / oxygen (O₂) is exchanged with the environment, as indicated at the top side of figure 1.

In the electricity generation process, a mixture of methane, steam and hydrogen is supplied to the solid oxide stack 1, while the solid oxide stack 1 is at a temperature in the above-mentioned range of temperatures of about 650°C to 750°C. As a result, the solid oxide stack 1 produces electricity and carbon dioxide. Steam and hydrogen are also output by the solid oxide stack 1. The methane is retrieved from the storage area, and hydrogen which originates from the solid oxide stack 1 is fed back to the solid oxide stack 1. The output steam is fed back to the steam source 4, and the carbon dioxide is fed back to the source 5 of carbon dioxide. The electricity is supplied to an electricity network 8. In the electricity generating process, air / oxygen is exchanged with the environment, as indicated at the top side of figure 2.

The solid oxide stack 1 is suitable to be used for enabling storage of electricity which is available at some time but cannot be used at that time, namely in the form of methane which is produced by means of the electricity. Hence, the solid oxide stack 1 can be functional to account for fluctuations in production of energy by various durable means including wind turbines as mentioned. In situations in which such means cannot produce all of the electricity as necessary to comply with a need for electricity, the solid oxide stack 1 can be used to generate electricity, in which process methane is taken from a storage area and supplied to the solid oxide stack 1.

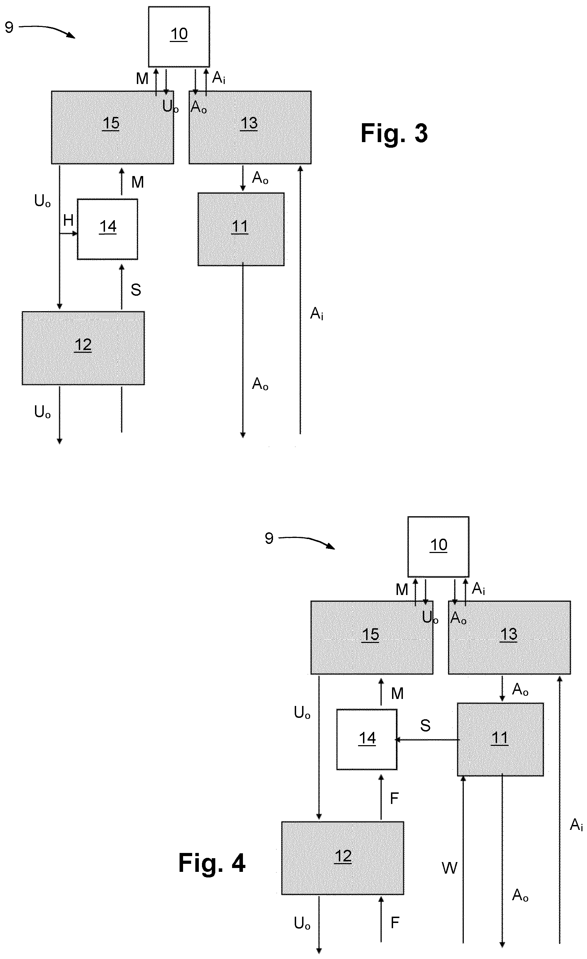

Figures 3 and 4 show a scheme of components of a module 9 including a solid oxide unit 10, wherein figure 3 illustrates use of the components in the methane production process, and wherein figure 4 illustrates use of the components in the electricity generation process. The solid oxide unit 10 comprises at least one solid oxide stack 1. A practical example of the number of solid oxide stacks 1 in the solid oxide unit 10 is four. The other components of the module 9 which are diagrammatically shown in figures 3 and 4 are the following:
- a first heat exchanger 11 arranged and configured to enable exchange of heat between output air Aₒ of the solid oxide unit 10 and water W originating from a water supply to the module 9 during the electricity generation process;
- a second heat exchanger 12 arranged and configured to enable exchange of heat between an output mixture Uₒ of the solid oxide unit 10, comprising steam and hydrogen, and gaseous fuel F comprising methane originating from a methane supply to the module 9 and hydrogen during the electricity generation process;
- a third heat exchanger 13 arranged and configured to enable exchange of heat between output air Aₒ of the solid oxide unit 10 and input air Aᵢ originating from an air supply to the module 9 during both the methane production process and the electricity generation process, and to pass the input air Aᵢ on to the solid oxide unit 10;

- a mixing and dosing unit 14 arranged and configured to receive steam S originating from a steam source 4 such as a vessel which is operable to produce steam, and to also receive output hydrogen H of the solid oxide unit 10 during the methane production process, to receive steam S from the first heat exchanger 11 and also the gaseous fuel F from the second heat exchanger 12 during the electricity generation process, and to output a mixed flow M during both the methane production process and the electricity generation process; and
- a fourth heat exchanger 15 arranged and configured to enable exchange of heat between the output mixture Uₒ of the solid oxide unit 10 and an output mixed flow M of the mixing and dosing unit 14 during both the methane production process and the electricity generation process, and to pass the output mixed flow M of the mixing and dosing unit 14 on to the solid oxide unit 10.

It follows from the foregoing that during the methane production process, among other things, input air Aᵢ is heated by output air Aₒ of the solid oxide unit 10 in the third heat exchanger 13, and the output mixed flow M of the mixing and dosing unit 14 is heated by the output mixture Uₒ of the solid oxide unit 10 in the fourth heat exchanger 15. Hence, discharge heat of the solid oxide unit 10 is not simply wasted, but is reused instead, which is beneficial to the efficiency of the module and ultimately to the costs of the electricity which is generated by the module in the electricity generation process. Reuse of discharge heat also takes place during the electricity generation process, as water W is heated by the output air Aₒ of the solid oxide unit 10 in the first heat exchanger 11, gaseous fuel F is heated by the output mixture Uₒ of the solid oxide unit 10 in the second heat exchanger 12, input air Aᵢ is heated by output air Aₒ of the solid oxide unit 10 in the third heat exchanger 13, and the output mixed flow M of the mixing and dosing unit 14 is heated by the output mixture Uₒ of the solid oxide unit 10 in the fourth heat exchanger 15.

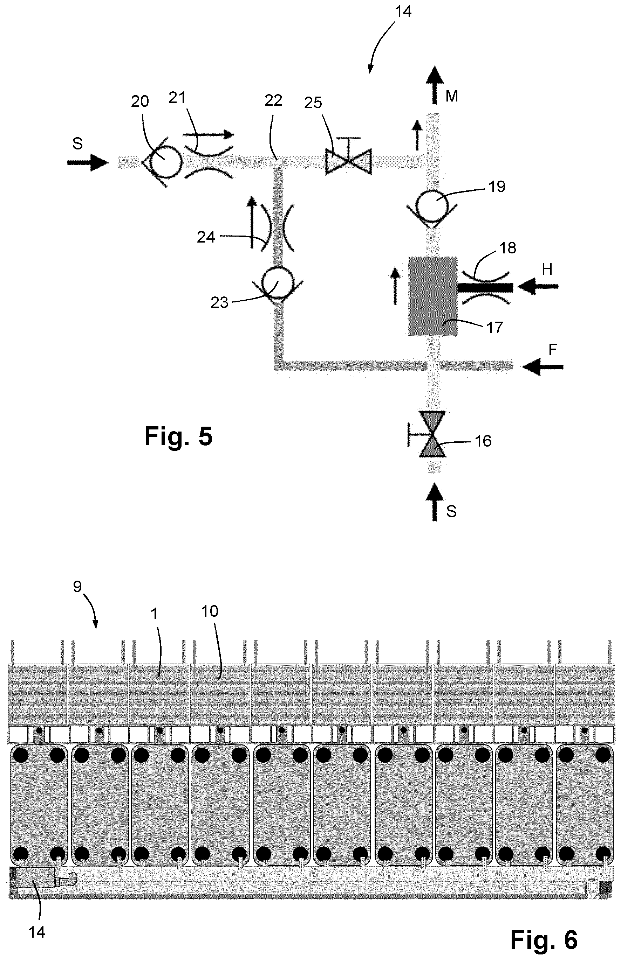

The mixing and dosing unit 14 is functional to supply the various gases as used by the solid oxide unit 10 in the methane production process and the electricity generation process, respectively, in relative amounts as beneficial to operation of the solid oxide unit 10. Practical examples of the output mixed flow M of the mixing and dosing unit 14 are a mixture of 90% steam and 10% hydrogen during the methane production process and a mixture of 48% steam, 25% hydrogen and 27% methane during the electricity generation process. To make sure that a gas mixture as envisaged is supplied to the solid oxide unit 10, several components can be used, as will now be explained with reference to figure 5, in which a practical configuration of the mixing and dosing unit 14 is illustrated.

During the methane production process, steam S flows through a valve 16, which is in an opened position in the methane production process and in a closed position in the electricity generation process, towards a venturi eductor 17. The amount of steam S passing through the venturi eductor 17 is controlled by means of a nozzle in the venturi eductor 17. As a result of underpressure which is created in this way, hydrogen H is sucked in and is mixed with the steam S. Dosing of the hydrogen H is done by means of an orifice 18 located in the supply of the hydrogen H. To avoid a gas flow in the wrong direction, a check valve 19 is located at an exit side of the venturi eductor 17.

During the electricity production process, steam S flows through a check valve 20 and an orifice 21, wherein use of the check valve 20 ensures a correct flow direction and use of the orifice 21 ensures proper dosing of the steam S. Both a configuration of the mixing and dosing unit 14 in which the steam S passes the check valve 20 first and the orifice 21 later and a configuration of the mixing and dosing unit 14 in which the steam S passes the orifice 21 first and the check valve 20 later are feasible. Gaseous fuel F comprising methane and hydrogen is supplied to the flow of steam S, at the position of a T piece 22, wherein also a check valve 23 and an orifice 24 are used, and wherein also both a configuration of the mixing and dosing unit 14 in which the gaseous fuel F passes the check valve 23 first and the orifice 24 later and a configuration of the mixing and dosing unit 14 in which the gaseous fuel F passes the orifice 24 first and the check valve 23 later are feasible. The mixture M obtained on the basis of the steam S and the gaseous fuel F flows through a valve 25, which is in an opened position in the electricity generation process and in a closed position in the methane production process, and exits the mixing and dosing unit 14 in the direction of the solid oxide unit 10.

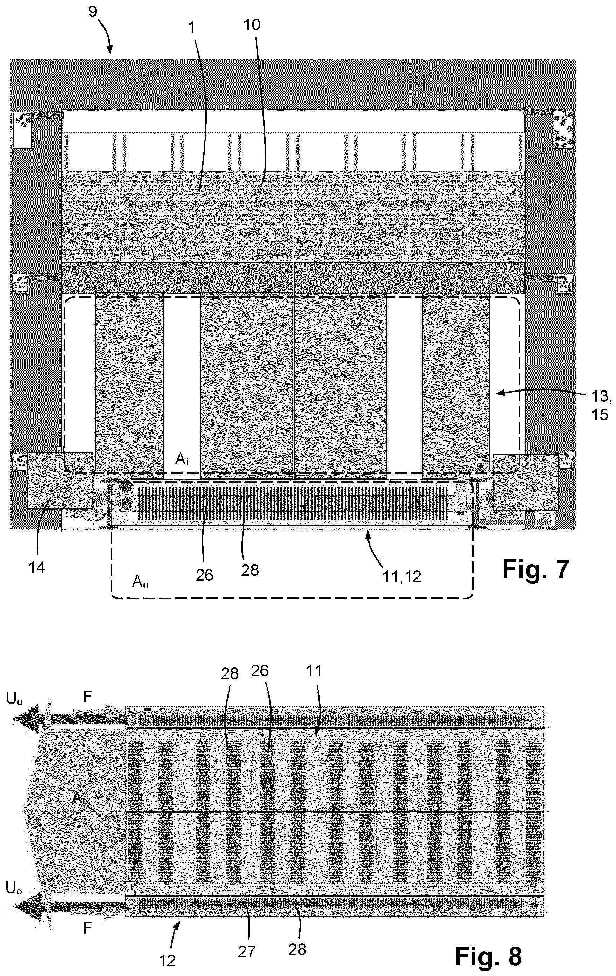

Figures 6 and 7 diagrammatically show a front view and a side view, respectively, of the module 9. Among other things, it can be seen that the module 9 comprises a number of solid oxide units 10. In the present example, the number is twenty. Further, in the present example, the module 9 comprises one first heat exchanger 11, two second heat exchangers 12, twenty third heat exchangers 13, two mixing and dosing units 14, and twenty fourth heat exchangers 15. The solid oxide units 10 are at a highest position, the first heat exchanger 11 and the second heat exchangers 12 are at a lowest position, and the third heat exchangers 13 and the fourth heat exchangers 15 are at an intermediate position. In figure 6, tubes for transporting input fluids and output fluids in both the methane production process and the electricity generation process are visible at a bottom side of the figure. In figure 7, respective areas of input air Aᵢ and output air Aₒ are depicted in dashed lining.

A practical example of a power level of the solid oxide cell 2 during the methane production process is 43 W. This implies that a power level of the solid oxide stack 1 comprising seventy solid oxide cells 2 is about 3 kW, that a power level of the solid oxide unit 10 comprising four solid oxide stacks 1 is about 12 kW, and that a power level of the module 9 comprising twenty solid oxide units 10 is about 240 kW. Having the module 9 is beneficial to the costs of the electricity which is generated during the electricity generation process, because a large, combined power level is combined with one system for supplying and discharging the necessary fluids and one system for reusing heat, which systems are suitable to be used in both the methane production process and the electricity generation process.

As suggested earlier, the solid oxide unit 10 is typically operated at a temperature which is in a range of about 650°C to 750°C, in order to obtain the best results. The first heat exchanger 11 and the second heat exchanger 12 are typically operated at a temperature which is in a range of about 120°C to 200°C, and the third heat exchanger 13 and the fourth heat exchanger 15 are typically operated at an intermediate temperature, i.e. a temperature which is in a range of about 120°C to 750°C. The mixing and dosing unit 14 is operated at a temperature which is in a range of about 80°C to 120°C. On the basis of these temperatures, a correct functioning of the various components of the module 9 is realized. The temperatures are also advantageous when it comes to the lifespan of the components. The positioning of the components in a height direction is chosen so as to be logical in view of the temperatures.

Figure 8 diagrammatically shows a top view of a section of the module 9 at the position of heat exchanger tubes extending in the module 9, including tubes of a tube system 26 of the first heat exchanger 11 which serve for transporting the water W during the electricity generation process, and tubes of a tube system 27 of the two second heat exchangers 12 which serve for transporting the gaseous fuel F comprising methane and hydrogen during the electricity generation process. For the purpose of promoting the exchange of heat between the water W in the tube system 26 of the first heat exchanger 11 and the output air Aₒ of the solid oxide unit 10 flowing through an area where the tube system 26 is located, part of the tube system 26 is provided with bodies 28 which are functional to increase a heat exchanging surface of the tube system 26, and which may also be referred to as fins, for example. Likewise, for the purpose of promoting the exchange of heat between the gaseous fuel F in the tube system 27 of the second heat exchangers 12 and the output mixture Uₒ of the solid oxide unit 10 flowing along the tube systems 27 in opposite flow direction, part of the tube system 27 is provided with bodies 28 which are functional to increase a heat exchanging surface of the tube system 27 as mentioned.

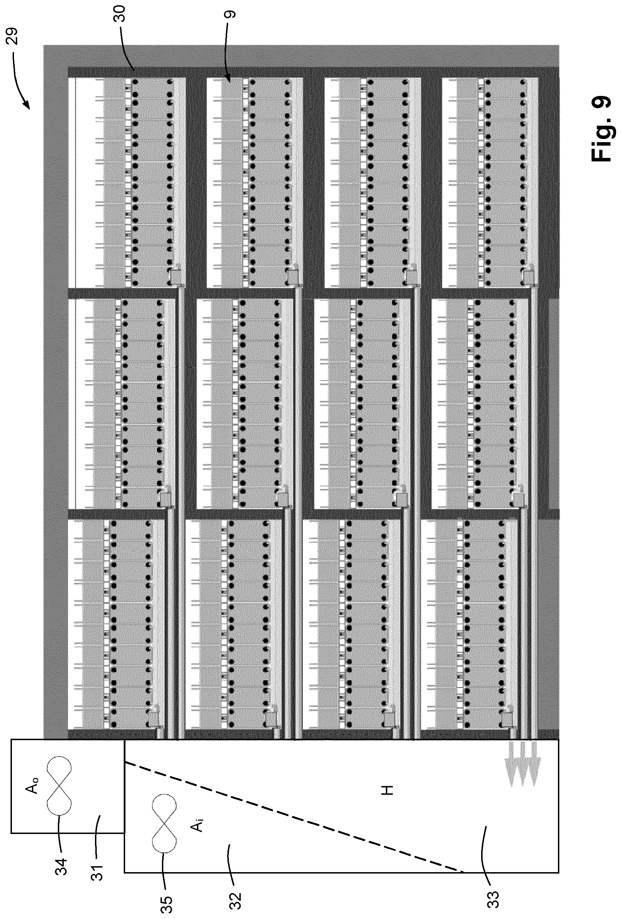

With reference to figure 9, it is noted that in order to further reduce the costs of the electricity, modules 9 are combined to an even larger entity, namely a subsystem 29. In the present example, the subsystem 29 comprises a frame 30 and twelve modules 9 arranged in the frame 30 in three columns of four modules 9. With reference to the earlier example value of the power level of the module 9, this implies that a power level of the subsystem 29 is close to 3 MW during the methane production process.

Besides the modules 9 and the frame 30, the module 9 comprises a single air discharge channel 31, which is shared by all of the modules 9 and in fluid communication with the modules 9 for receiving air Aₒ from the modules 9 during both the methane production process and the electricity generation process, a single air supply channel 32, which is shared by all of the modules 9 and in fluid communication with the modules 9 for supplying air Aᵢ to the modules 9 during both the methane production process and the electricity generation process, and a single hydrogen discharge channel 33, which is shared by all of the modules 9 and in fluid communication with the modules 9 for receiving hydrogen H from the modules 9 during the methane production process. The respective shared channels 31, 32, 33 of the subsystem 29 are diagrammatically shown at the left side of figure 9. In the air discharge channel 31, suction means 34 such as a fan are present to actively extract air Aₒ at the position of the air discharge channel 31. Likewise, in the air supply channel 32, blower means 35 such as a fan are present to exert pressure on the air Aᵢ and to put the air Aᵢ in motion towards the modules 9 at the position of the air supply channel 32.

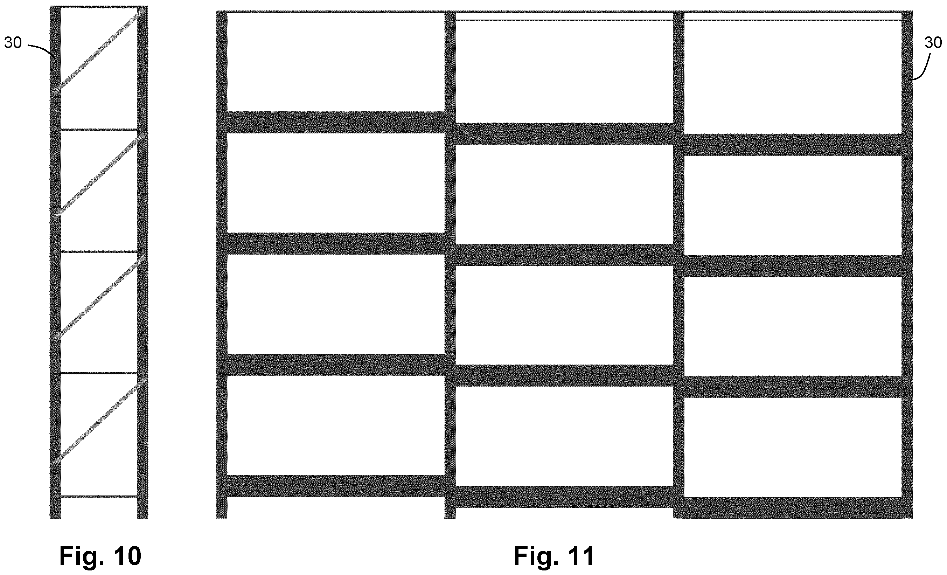

The frame 30 is separately shown in figures 10 and 11. A notable feature of the subsystem 29 is that the frame 30 is designed to have a staggered configuration of the columns of the modules 9. This enables a configuration in which generally straight tube systems extend from the respective columns of modules 9 to the one side of the subsystem 29 where the shared channels 31, 32, 33 are located without being in the way of each other. In this respect, it may be so that respective tube systems are in stacked configuration in column direction. This is indicated in figure 9 by means of three arrows indicating three output flows of the three columns.

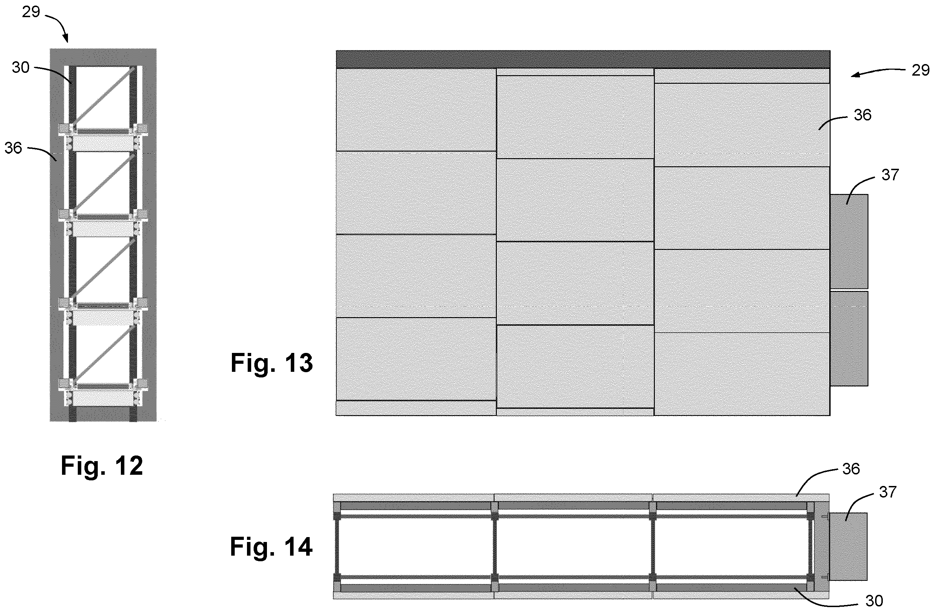

Advantageously, thermal insulation measures are taken in order to preserve heat in the subsystem 29 as much as possible. In figures 12, 13 and 14, it is illustrated that in this respect, it is practical if thermal insulation sheets 36 are used for covering the subsystem 29. The thermal insulation sheets 36 can be fixed to the frame 30 in any suitable way and by any suitable means, wherein it is possible that the thermal insulation sheets 36 are removably coupled to the frame 30. In figures 13 and 14, it is further shown that the subsystem 29 may be equipped with boxes 37 for accommodating electronic components including components which are functional to control operation of the subsystem 29.

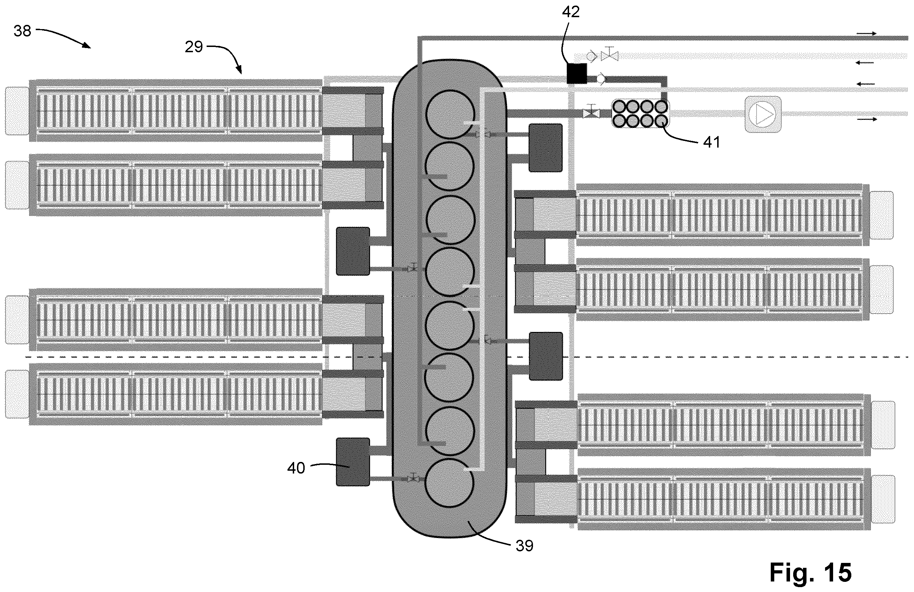

With reference to figure 15, it is noted that in order to even further reduce the costs of the electricity, subsystems 29 are combined to an even larger entity, namely a system 38. In the present example, the system 38 comprises eight subsystems 29. With reference to the earlier example value of the example value of the modules 9, this implies that a power level of the system 38 is about 23 MW. The system 38 comprises a single steam vessel 39, which is shared by all of the modules 9 of the subsystems 29. In the present example, the eight subsystems 29 of the system 38 are arranged in two groups of four subsystems 29 at opposite sides of the steam vessel 39. Using a shared steam vessel 39 in a system 38 having a power level of about 23 MW is advantageous as far as the costs of the electricity to be output by the system 38 are concerned. Examples of further cost-saving measures are using one hydrogen compressor 40 per two subsystems 29, using one set 41 of membrane filters in the system 38 for separating hydrogen from carbon dioxide, and using one mix ratio module 42 in the system 38 for supplying the gaseous fuel F in an appropriate amount and with appropriate mixture percentages.

Examples of dimensions of the module 9 are as follows: a width may be about 2.2 meters, a depth may be about 1.4 meters, and a height may be about 1 meter. Examples of dimensions of the subsystem 29 are as follows: a width may be about 7 meters, a depth may be about 1.4 meters, and a height may be about 5 meters.

It will be clear to a person skilled in the art that the scope of the invention is not limited to the examples discussed in the foregoing, but that several amendments and modifications thereof are possible without deviating from the scope of the invention as defined in the attached claims. It is intended that the invention be construed as including all such amendments and modifications insofar they come within the scope of the claims or the equivalents thereof. While the invention has been illustrated and described in detail in the figures and the description, such illustration and description are to be considered illustrative or exemplary only, and not restrictive. The invention is not limited to the disclosed embodiments. The figures are schematic, wherein details which are not required for understanding the invention may have been omitted, and not necessarily to scale.

Notable aspects of the invention as described in the foregoing are generally defined as follows. In the field of using methane as an energy storage medium, wherein electricity, which may be excess electricity in a non-fossil system, is used at one stage for producing methane, and wherein methane is used at a later stage for generating electricity, when more electricity is needed than can be generated by the non-fossil system at that later stage, use is made of solid oxide cells 2. The solid oxide cells 2 are combined to solid oxide stacks 1, the solid oxide stacks 1 are arranged in solid oxide units 10, and the solid oxide units 10 are arranged in modules 9. Each of the modules 9 is included in a subsystem 29 and is arranged in a frame 30 of the subsystem 29. Finally, a number of subsystems 29 are combined to a system 38. In this way, costs of the electricity to be generated are reduced. Further reduction of the costs is achieved by having components which are shared between the modules 9 and the subsystems 29, particularly components involved in supplying and discharging fluids and components involved in reusing heat of output fluid flows.

## Claims

1. Module (9) configured for application in a methane production process comprising use of electricity in producing methane on the basis of steam and carbon dioxide, and also for application in an electricity generation process comprising use of methane in generating electricity and producing carbon dioxide and steam, the module (9) comprising:
- a solid oxide unit (10) comprising at least one solid oxide stack (1) of at least two solid oxide cells (2), wherein each of the solid oxide cells (2) comprises a component configured to function as anode during operation of the solid oxide cell (2), a component configured to function as cathode during operation of the solid oxide cell (2), and a solid oxide component configured to function as electrolyte during operation of the solid oxide cell (2);
- a first heat exchanger (11) arranged and configured to enable exchange of heat between output air (Aₒ) of the solid oxide unit (10) and water (W) originating from a water supply to the module (9) during the electricity generation process;
- a second heat exchanger (12) arranged and configured to enable exchange of heat between an output mixture (Uₒ) of the solid oxide unit (10), comprising steam and hydrogen, and gaseous fuel (F) comprising methane originating from a methane supply to the module (9) and hydrogen during the electricity generation process;
- a third heat exchanger (13) arranged and configured to enable exchange of heat between output air (Aₒ) of the solid oxide unit (10) and input air (Aᵢ) originating from an air supply to the module (9) during both the methane production process and the electricity generation process, and to pass the input air (Aᵢ) on to the solid oxide unit (10);
- a mixing and dosing unit (14) arranged and configured to receive steam (S) originating from a steam source (4, 39) and also output hydrogen (H) of the solid oxide unit (10) during the methane production process, to receive steam (S) from the first heat exchanger (11) and also the gaseous fuel (F) from the second heat exchanger (12) during the electricity generation process, and to output a mixed flow (M) during both the methane production process and the electricity generation process; and
- a fourth heat exchanger (15) arranged and configured to enable exchange of heat between the output mixture (Uₒ) of the solid oxide unit (10) and an output mixed flow (M) of the mixing and dosing unit (14) during both the methane production process and the electricity generation process, and to pass the output mixed flow (M) of the mixing and dosing unit (14) on to the solid oxide unit (10).

2. Module (9) as claimed in claim 1, wherein the first heat exchanger (11) comprises a tube system (26) for transport of water (W) and creation of steam (S), and wherein bodies (28) which are functional to increase a heat exchanging surface of the tube system (26) are mounted on at least a portion of the tube system (26).

3. Module (9) as claimed in claim 1 or 2, wherein the second heat exchanger (12) comprises a tube system (27) for transport of the gaseous fuel (F), and wherein bodies (28) which are functional to increase a heat exchanging surface of the tube system (27) are mounted on at least a portion of the tube system (27).

4. Module (9) as claimed in any of claims 1-3, wherein the hydrogen in the gaseous fuel (F) originates from the solid oxide unit (10).

5. Module (9) as claimed in any of claims 1-4, wherein the output mixed flow (M) of the mixing and dosing unit (14) comprises steam and hydrogen during the methane production process and steam, hydrogen and methane during the electricity generation process.

6. Module (9) as claimed in any of claims 1-5, comprising a catalyst unit (6) including a catalyst, wherein the catalyst unit (6) is arranged and configured to receive a mixture of hydrogen originating from the solid oxide unit (10) and carbon dioxide during the methane production process, and to enable interaction between the catalyst and the mixture of hydrogen and carbon dioxide.

7. Subsystem (29) comprising a frame (30) and at least two modules (9) as claimed in any of claims 1-6, wherein the modules (9) are arranged in the frame (30).

8. Subsystem (29) as claimed in claim 7, wherein the frame (30) is shaped like a matrix and is designed to position at least four modules (9) in at least two columns.

9. Subsystem (29) as claimed in claim 8, wherein the frame (30) is designed to have a staggered configuration of the columns of the modules (9).

10. Subsystem (29) as claimed in claim 9, wherein tube systems for discharging gas from a row of modules (9) are generally straight and are in stacked configuration in column direction.

11. Subsystem (29) as claimed in any of claims 7-10, comprising a single air discharge channel (31) in fluid communication with the modules (9) for receiving air (Aₒ) from the modules (9) during both the methane production process and the electricity generation process, and suction means (34) to actively extract air (Aₒ) at the position of the air discharge channel (31).

12. Subsystem (29) as claimed in any of claims 7-11, comprising a single air supply channel (32) in fluid communication with the modules (9) for supplying air (Aᵢ) to the modules (9) during both the methane production process and the electricity generation process, and blower means (35) to exert pressure on the air (Aᵢ) and to put the air (Aᵢ) in motion towards the modules (9) at the position of the air supply channel (32).

13. Subsystem (29) as claimed in any of claims 7-12, comprising a single hydrogen discharge channel (33) in fluid communication with the modules (9) for receiving hydrogen (H) from the modules (9) during the methane production process.

14. System (38) comprising at least two subsystems (29) as claimed in any of claims 7-13.

15. System (38) as claimed in claim 14, comprising a single steam vessel (39) in fluid communication with the modules (9) of the subsystems (29) for supplying steam (S) to the modules (9) of the subsystems (29) during the methane production process.
